# EUROPÄISCHE PATENTSCHRIFT

(11) **EP 2 521 114 B1**
(45) Veröffentlichungstag und Bekanntmachung des Hinweises auf die Patenterteilung: **06.12.2017**
(21) Anmeldenummer: 12165536.9
(22) Anmeldetag: 25.04.2012
(51) Int. Cl.: G09F 3/10, G09F 3/14, A61M 5/14, A61J 1/10, A61J 1/14, A61J 1/18, B65D 23/00, G09F 3/02

(54) **Aufhängeetikett für einen Flüssigkeitsbeutel und Verfahren zum Anbringen eines Etiketts an einem mit Flüssigkeit gefüllten Beutel**
Hanger label for a fluid bag and method for applying a label to a bag filled with liquid
Etiquette de suspension pour un sachet de liquide et procédé d'application d'une étiquette sur un sachet rempli de liquide

(30) Priorität: 02.05.2011 DE 102011100156
(43) Veröffentlichungstag der Anmeldung: 07.11.2012
(73) Patentinhaber: Schreiner Group GmbH & Co. KG, 85764 Oberschleissheim (DE)
(72) Erfinder: Seidl, Peter, 81371 München (DE)
(74) Vertreter: Epping - Hermann - Fischer

(56) Entgegenhaltungen:
- GB-A- 1 032 043
- US-A- 5 782 495
- US-B1- 6 457 747

## Beschreibung

Die vorliegende Erfindung betrifft ein Etikett für einen mit einer Flüssigkeit gefüllten Beutel sowie ein Verfahren zur Befestigung eines solches Etiketts an einem entsprechenden Beutel. Insbesondere betrifft die vorliegende Erfindung ein Etikett für einen Beutel, wie beispielsweise einen Infusions- oder Transfusionsbeutel, wobei das Etikett möglichst sicher an dem Beutel fixiert werden kann, damit eine sichere Aufhängung des etikettierten Beutels ermöglicht wird.

Für die Verabreichung spezieller Medikamente und pharmazeutischer Produkte kann es erforderlich sein, dass ein Wirkstoff in einem Flüssigkeitsvolumen, beispielsweise einer Kochsalzlösung, aufbereitet wird. Diese Lösung kann dann einem Patienten mittels einer Infusion verabreicht werden. Hierzu muss die fertig aufbereitete Lösung in ein Behältnis abgefüllt werden. Dieses Behältnis wird dann zur Verabreichung in der Nähe des Patienten aufgehängt und die Flüssigkeit gelangt über einen Schlauch mit einer Infusionsnadel in den Blutkreislauf des Patienten.

Vorzugsweise werden als Behältnisse für eine solche pharmazeutische Lösung Glasflaschen oder Kunststoffbeutel verwendet. Bei Glasflaschen besteht dabei jedoch die Gefahr, dass diese bei mechanischer Beanspruchung leicht zerstört werden können. Dabei besteht dann für die behandelnde Person die Gefahr, mit der aufbereiteten Lösung in Berührung zu kommen was beispielsweise bei Zytostatika eine große Gefahr darstellt. Außerdem stellen auch die Glassplitter einer zerbrochenen Glasflasche mit ihren scharfen Kanten ein erhebliches Verletzungsrisiko dar.

Daher wird für Bereitstellung von Infusionslösungen vermehrt auf Kunststoffbeutel zurückgegriffen. Im Gegensatz zu Glas besteht jedoch bei Kunststoffbeuteln die Gefahr, dass gewisse chemische Substanzen durch den Beutel hindurch diffundieren können und so die pharmazeutische Lösung verunreinigt würden. Diese Gefahr besteht insbesondere dann, wenn zur Kennzeichnung eines Beutels auf den Beutel ein Etikett aufgeklebt wird und dann bestimmte, leicht flüchtige Bestandteile des Klebstoffes auf dem Etikett durch den Beutel hindurchdiffundieren. Dieses Phänomen wird als Klebstoffmigration bezeichnet.

Darüber hinaus stellt sich auch gerade bei relativ kleinen Beuteln das Problem, diese in ausreichendem Maße zu kennzeichnen, da kleinere Beutel zwangsläufig auch eine relativ kleine Oberfläche besitzen, auf der ein Etikett aufgebracht werden könnte. Andererseits ist für die Kennzeichnung des Medikaments mit allen erforderlichen Warnhinweisen und Dokumentationselementen eine relativ ausführliche und große Fläche erforderlich.

Ferner wird durch das Aufkleben eines Etiketts auf den üblicherweise transparenten Beutel der Inhalt des Beutels durch das Etikett verdeckt. Somit können eventuelle vorhandene Verunreinigungen oder auch ungewöhnliche Verfärbungen des Inhalts optisch nicht mehr wahrgenommen und entdeckt werden.

Aus den oben genannten Gründen ist daher ein Bekleben eines Beutels mit einem konventionellen Etikett mit Nachteilen und Risiken verbunden. Andererseits kann auf die Kennzeichnung eines Infusionsbeutels auf keinen Fall verzichtet werden. Vielmehr ist es gerade in kritischen Situationen unabdingbar, dass eine behandelnde Person innerhalb kürzester Zeit sich mit dem Inhalt des vorliegenden Infusionsbeutels vertraut machen kann und den Inhalt vor der Verabreichung sorgfältig überprüft.

In der US 6 457 747 B1 ist ein Etikett zum Aufhängen eines Behältnisses beschrieben. Das Etikett weist eine Reihe von Einstanzungen auf, so dass ein Bügel gebildet wird, wenn an einer Anfasslasche angezogen wird. Der Bügel ist an seinen Endpunkten mit dem auf dem Behältnis aufgeklebten Etikett verbunden. Der Bügel ist dazu vorgesehen, an einem Holm aufgehängt zu werden. Das Etikett besteht aus mehreren Schichten, die durch eine Klebstoffschicht ihrerseits miteinander verbunden sind.

Es ist daher eine Aufgabe der vorliegenden Erfindung ein Etikett zur Kennzeichnung solcher Infusionsbeutel bereitzustellen, das einerseits eine ausreichende Kennzeichnung des Beutels ermöglicht und andererseits die oben genannten Probleme wie beispielsweise Klebstoffmigration oder das Verdecken des Inhalts vermeidet.

Außerdem ist es eine Aufgabe der vorliegenden Erfindung ein effizientes und möglichst automatisches Etikettieren eines Infusionsbeutels mit einem erfindungsgemäßen Etikett zu ermöglichen.

Dies wird durch ein Etikett für einen mit einer Flüssigkeit gefüllten Beutel erzielt, das eine erste Folie umfasst, wobei die erste Folie eine Unterseite und eine zumindest teilweise mit Klebstoff beschichtete Oberseite besitzt. Das Etikett umfasst ferner eine zweite Folie mit einer Oberseite und einer Unterseite, wobei die Unterseite der ersten Folie zumindest teilweise fest mit der Oberseite der zweiten Folie verbunden ist, um einen Folienverbund zu bilden. Der Folienverbund aus erster Folie und zweiter Folie besitzt eine Einstanzung aus einem offenen Linienzug mit zwei Endpunkten, so dass eine streifenförmige Lasche entsteht. Die streifenförmige Lasche ist entlang einer gedachten Linie zwischen den beiden Endpunkten mit dem übrigen Folienverbund verbunden. Die streifenförmige Lasche ist eingerichtet, durch eine in einem Beutel befindlichen Öffnung geführt zu werden, um die streifenförmige Lasche mit einem umgeklappten Teil des Etiketts zu verkleben. Es sind eine weitere erste und eine weitere zweite Einstanzung jeweils aus einem offenen oder einem geschlossenen Linienzug durch die erste und durch die zweite Folie eingerichtet zum Aufhängen des Etiketts vorhanden.

Die Erfindung umfasst ferner ein Verfahren zum Anbringen eines Etiketts an einem mit einer Flüssigkeit gefüllten Beutel, bei dem zunächst das Etikett aufgenommen wird. Daraufhin wird die streifenförmige Lasche aufgestellt und diese durch eine Öffnung in dem Beutel hindurchgeführt. Anschließend wird mindestens ein Teilbereich der Oberseite der ersten Folie mit mindestens
einem Teilbereich des Beutels verklebt. Ferner wird die aufgestellte streifenförmige Lasche umgeklappt.

Es ist ein besonderer Ansatz der vorliegenden Erfindung, einen zweilagigen Folienaufbau so auszugestalten, dass aus dem Folienverbund heraus eine Lasche entsteht. Diese Lasche kann dann durch eine Aufhängeöffnung des Beutels hindurchgeführt und umgeschlagen werden. Durch diese spezielle Konstruktion ist es ausreichend, dass der Folienverbund für einen sicheren Halt nur an einem oberen Rand des Beutels befestigt werden muss. Da der Bereich dieses oberen Rands in der Regel von dem darunter befindlichen Flüssigkeitsreservoir abgetrennt ist und somit keine Flüssigkeit enthält, wird somit die Gefahr einer Klebstoffmigration in das im Beutel enthaltene Medikament ausgeschlossen. Ferner wird, da das Etikett nur in einem kleinen Teilbereich des Beutels aufgebracht wird, der Inhalt des Beutels nicht verdeckt und eine eventuelle Verunreinigung kann sofort erkannt werden.

Für die Etikettierung eines Beutels mit dem erfindungsgemäßen Etikett ist es dabei von Vorteil, dass die streifenförmige Lasche die Neigung beziehungsweise die Eigenschaft besitzt, in die Ausgangslage zurückzukehren. Somit bildet sich eine Art rückfederndes Gelenk aus. Dieser Effekt wird dadurch erzielt, dass der Folienverbund aus zwei einzelnen Folienlagen gebildet wird und darüber hinaus am Punkt des Gelenkes geeignete Einstanzungen angebracht werden. Insbesondere für die effiziente und automatisierte Etikettierung eines Beutels sind die besonderen Eigenschaften, die mit der speziellen Lasche verbunden sind, von Vorteil, da die Lasche einerseits eine relativ hohe Steifigkeit besitzt und andererseits durch die Gelenkfunktion ein klar definiertes Herausschwenken aus der Folienebene ermöglicht wird.

In einer Ausführungsform ist die Unterseite der ersten Folie zumindest teilweise fest mit der Oberseite der zweiten Folie verklebt ist. Somit ergibt sich ein besonders stabiler Folienverbund.

Vorzugsweise ist die Oberseite der zweiten Folie teilweise mit einer Klebstoff abweisenden Substanz versehen und die erste Folienlage umfasst zusätzlich in dem Bereich, der auf der zweiten Folienlage zu liegen kommt, mindestens eine weitere Einstanzung, wobei die erste Folie so auf der auf der zweiten Folie zu liegen kommt, dass sich die weitere Einstanzung im Bereich der Klebstoff abweisenden Substanz befindet. Auf diese Weise können die Folienteile der ersten Folienlage leicht von dem Folienverbund abgenommen werden und als sogenannte Hilfs- oder Zusatzetiketten für Dokumentationszwecke verwendet werden.

Vorzugsweise besitzt der Folienverbund aus erster und zweiter Folie eine Einstanzung zum Aufhängen des Etiketts. Damit kann der Beutel an dieser Öffnung leicht an einer geeigneten Aufhängevorrichtung aufgehängt werden. Da das Etikett aufgrund der durch die Öffnung des Beutels geführten Lasche sehr fest mit dem Beutel verbunden ist, wird auch die Gefahr, dass sich das Etikett im aufgehängten Zustand vom Beutel löst, nahezu ausgeschlossen.

In einer speziellen Ausführungsform besitzt die erste Folie eine erste Dicke und die zweite Folie eine zweite Dicke, wobei die erste Dicke größer ist als die zweite Dicke. Mit diesem Folienverbund kann die Feder- und Gelenkwirkung der Lasche besonders gut realisiert werden.

Vorzugsweise umfasst mindestens die erste Folie ferner mindestens eine Einstanzung in der Nähe der Endpunkte des Linienzugs der Einstanzung der streifenförmigen Lasche. Somit werden die Gelenkeigenschaften des Folienverbundes noch weiter verbessert, was eine automatisierte Applikation des Etiketts begünstigt.

Vorzugsweise ist mindestens der Bereich der mit Klebstoff beschichteten Oberseite der ersten Folie mit einem bahnförmigen Material abgedeckt. In einer weiter bevorzugten Ausführungsform ist eine Mehrzahl von Etiketten auf einem bahnförmigen Material aufgebracht und das bahnförmige Material mit der Mehrzahl von Etiketten ist auf eine Rolle aufgewickelt. Somit können die Etiketten für die Weiterverarbeitung in besonders geeigneter Form bereitgestellt werden.

Vorzugsweise wird die Oberseite der ersten Folie mit dem Beutel nur in einem Bereich verklebt, in dem sich keine Flüssigkeit befindet. Somit kann die Gefahr von Klebstoffmigration nahezu vollständig eliminiert werden.

In einer speziellen Ausführungsform wird der Klebstoff des Etiketts nach dem Verkleben noch in einem weiteren Schritt ausgehärtet, so dass nach der Aushärtung eine besonders feste Verbindung zwischen Etikett und Beutel entsteht.

In einer besonderen Ausführungsform wird die Oberseite der ersten Folie so mit dem Beutel verklebt, dass die verklebte streifenförmige Lasche von einem Teilbereich der ersten Folie überdeckt wird. Auf diese Weise wird die Lasche zusätzlich fixiert und eine besonders zuverlässige Verbindung zwischen Etikett und Beutel erreicht.

Im Folgenden wird die Erfindung unter Bezugnahme auf die Figuren beschrieben. Dabei zeigen:
- Figur 1: einen schematischen Querschnitt durch ein erfindungsgemäßes Etikett;
- Figur 2: eine schematische Draufsicht auf ein erfindungsgemäßes Etikett;
- Figur 3: eine weitere schematische Draufsicht auf ein erfindungsgemäßes Etikett;
- Figur 4: eine schematische Darstellung eines Etiketts, wie es von einer Applikationsplatte aufgenommen wurde; und
- Figur 5: eine schematische Darstellung des Querschnitts eines Etikettes, das mit einem Flüssigkeitsbeutel verbunden wurde.

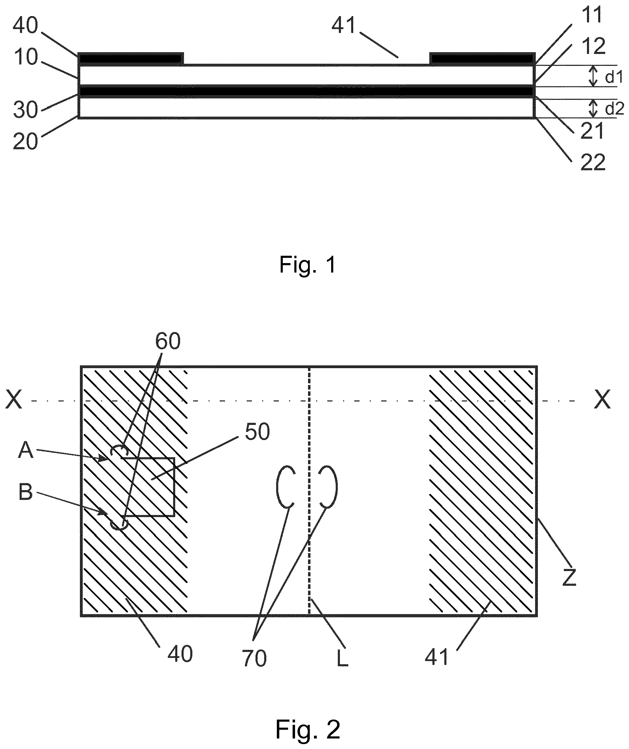

Figuren 1 und 2 zeigen schematische Darstellungen eines erfindungsgemäßen Etiketts 1. Figur 2 zeigt dabei eine Draufsicht auf das Etikett 1 und Figur 1 einen Querschnitt entlang der Schnittlinie X-X aus Figur 2.

Der Folienverbund umfasst oder besteht dabei aus einer ersten Folienlage 10 und einer zweiten Folienlage 20. Die beiden Folienlagen 10 und 20 sind dabei zumindest teilweise fest miteinander verbunden. Beispielsweise kann die Unterseite 12 der ersten Folienlage 10 und die Oberseite 21 der zweiten Folienlagen die beiden Folienlage 20 mit einem geeigneten Klebstoff 30 miteinander verklebt werden. Aber auch andere Arten, die beiden Folienlagen miteinander zu verbinden, insbesondere eine Folienlage auf die andere zu laminieren, sind möglich.

Auf die Oberseite 11 der ersten Folienlage 10 ist in Teilbereichen ein Haftklebstoff 40, 41 aufgetragen. Beispielsweise kann dieser Haftklebstoff 40, 41 in einem Druckverfahren auf die Oberseite 11 der ersten Folienlage 10 aufgedruckt werden.

Wie in Figur 2 dargestellt, ist in das Etikett eine streifenförmige Lasche 50 eingestanzt. Die Lasche 50 wird dabei durch eine Einstanzung in Form eines offenen Linienzugs zwischen den Endpunkten A und B gebildet. Die Einstanzung für die Lasche 50 geht dabei durch beide Folien 10 und 20 hindurch. Somit bildet sich entlang einer gedachten Linie zwischen beiden Punkten A und B ein Gelenk zwischen der Lasche 50 und dem restlichen Etikett 1 aus.

Darüber hinaus sind der oberen Folienlage 10 in der Nähe der Endpunkte A und B der Lasche 50 eine oder mehrere Einstanzungen 60 angebracht. Diese Einstanzungen 60 sind in Form und Größe auf die gewünschten Gelenkeigenschaften abgestimmt und tragen somit wesentlich zur Verbesserung der Gelenkfunktion bei. Insbesondere kann dadurch erreicht werden, dass beim Herausrücken der Lasche 50 aus dem Folienverbund die Lasche die Neigung besitzt, in ihre ursprüngliche Lage zurückzukehren.

Im linken Bereich des Etiketts 1 ist außerdem auf der Oberseite 11 der oberen Folienlagen 10 ein Klebstoff 40, 41 aufgetragen. Insbesondere ist der Klebstoff auch im Bereich der Lasche 50 aufgebracht.

Weiterhin kann das Etikett 1 noch Einstanzungen 70 aus einem offenen oder geschlossenen Linienzug enthalten. Diese Einstanzungen 70 sind so ausgestaltet, dass sie als Aufhängeöffnung dient. Die Einstanzungen 70 gehen durch beide Folienlagen 10 und 20 hindurch. Somit ist es möglich, beispielsweise einen Haken durch die Öffnung durchzuführen und daran das Etikett 1 gemeinsam mit dem etikettierten Beutel aufzuhängen.

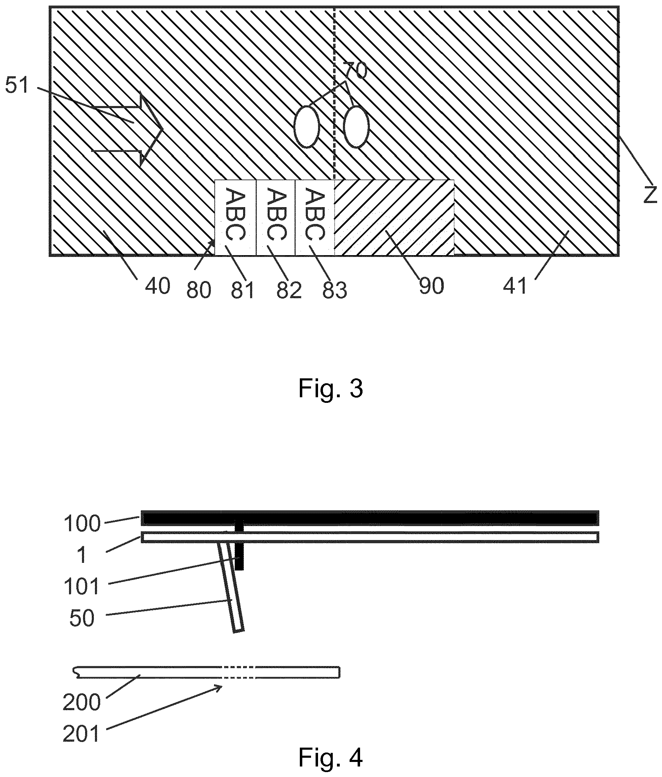

In einer speziellen Ausführungsform kann insbesondere die erste Folienlage 10 noch weitere Einstanzungen 80 umfassen, durch die Teilbereiche der ersten Folienlagen 10 abgetrennt sind. Die so abgetrennten Teilbereiche 81, 82 und 83 können dabei als abnehmbare Hilfs- oder Belegetiketten dienen, die zu einem späteren Zeitpunkt abgenommen und beispielsweise in der Patientenakte eingeklebt werden können. Zur besseren Darstellung ist in Figur 3 die Schraffierung 40 zur Kennzeichnung des Klebstoffauftrags in den Bereichen 81 bis 83 nicht eingezeichnet. Dennoch sind die Hilfs- oder Belegetiketten auf ihrer Unterseite mit einem Klebstoff 40 versehen.

Für das Ablösen dieser zusätzlichen Teiletiketten 81, 82 und 83 ist es dabei vorteilhaft, wenn die Oberseite 21 der zweiten Folienlagen 20 im Bereich der Teiletiketten mit einer klebstoffabweisenden Beschichtung 90 versehen ist. Beispielsweise kann hierzu auf die Oberseite 21 eine Silikonisierung aufgedruckt werden. Somit können die einzelnen Teiletiketten leicht abgenommen werden. Der Klebstoff 30 zwischen der ersten Folienlagen 10 und der zweiten Folienlagen 20, genauer auf der Unterseite 12 der ersten Folienlage, dient dabei den Teiletiketten 81, 82 und 83 gleichzeitig als Klebstoff für das spätere Einkleben der Teiletiketten in eine Patientenakte oder dergleichen.

Nach der Herstellung eines solchen Etiketts kann zumindest der mit dem Klebstoff 40, 41 versehene Bereich auf der Oberseite 11 der ersten Folienlage 10 mit einer Abdeckfolie versehen und somit geschützt werden. Insbesondere eignet sich hierzu ein sogenannter Liner, der auf der dem Klebstoff 40, 41 zugewandten Seite eine Klebstoff abweisenden Beschichtung aufweist.

Vorzugsweise werden auf einen solchen bahnförmigen Liner mehrere erfindungsgemäße Etiketten nebeneinander angeordnet. Somit kann der bahnförmige Liner mit den Etiketten einfach aufgerollt und für eine Weiterverarbeitung bereitgestellt werden.

Zur Etikettierung eines Infusionsbeutels mit einem solchen Etikett kann ein einzelnes Etikett zunächst vor dem Liner abgenommen werden. Beispielsweise kann das Etikett mit einer Saugplatte abgelöst werden. Figur 4 zeigt eine solche Platte 100, an deren Unterseite sich ein erfindungsgemäßes Etikett 1 angesaugt.

Die Platte 100 besitzt hierbei ferner einen Dorn 101 oder dergleichen. Dieser Dorn 101 drückt bei der Aufnahme des Etiketts 1 auf der Platte 100 die Lasche 50 aus der Folienebene heraus. Idealerweise stellt sich die Lasche 50 dabei nahezu senkrecht auf, wie dies exemplarisch in Figur 4 dargestellt ist. Aber auch ein anderer Aufstellwinkel der Lasche 50 ist durchaus akzeptabel.

Für das Hindurchführen der Lasche 50 durch eine Öffnung im Beutel ist es dabei besonders vorteilhaft, wenn am aufgestellten Ende der Lasche eine Spitze ausgebildet ist. Beispielsweise kann das freie Ende der Lasche 51 eine pfeilförmige Ausgestaltung besitzen, wie dies in Figur 3 dargestellt ist.

Ferner hat es sich als vorteilhaft erwiesen, wenn für den Aufbau des Folienverbundes zwei Folien mit unterschiedlicher Dicke verwendet werden. Dabei ergeben sich besonders gute Gelenkeigenschaften, wenn die Folie für die erste Folienlage 10 eine größere Dicke d1 besitzt, als die Folie für die zweite Folienlage 20. Folienverbunde, in denen die erste Folienlage eine Dicke d1 von 100 bis 200 Mikrometer aufweist sind hierfür gut geeignet. Für die zweite Folienlage haben sich Folien mit einer Dicke d2 zwischen 36 und 100 Mikrometer als geeignet herausgestellt.

Vorzugsweise werden für beide Folienlagen Folien aus Polyester verwendet. Aber auch Folienverbunde, bei denen eine oder beide Folienlagen aus einem anderen Material bestehen, sind ohne weiteres möglich.

Aufgrund des zweilagigen Folienverbundes und auch der zusätzlichen Einstanzungen 60 bildet sich an dem mit dem restlichen Etikett verbundenen Ende der Lasche ein Gelenk aus. Die Lasche 50 selbst dagegen besitzt eine ausreichende Steifigkeit und behält auch beim Herausdrücken der Lasche aus der Folienebene ihre Form. Durch die spezielle zweilagige Ausgestaltung und / oder die zusätzlichen Einstanzungen 60 wird erreicht, dass sich die Lasche 50 in wohldefinierter Art herausdrücken lässt.

Ein wie oben vorbereitetes Etikett mit aufgestellter Lasche 50 kann nun an einem Infusionsbeutel 200 angebracht werden. Dazu wird das auf der Platte 100 befindliche Etikett 1 so mit dem Beutel 200 zusammengeführt, dass die herausgedrückte Laschen 50 durch eine Aufhängeöffnung 201 des Beutels 200 hindurchgeführt wird. Anschließend klappt die Lasche 50 auf der sich dem Etikett gegenüber befindenden Seites des Beutels 200 in ihre ursprüngliche Lage zurück. In einem nachfolgenden Schritt wird das Etikett 1 entlang einer gedachten Linie L umgeklappt und der zweite Endbereich Z, der dem bereits mit dem Beutel 200 verklebten ersten Endbereich gegenüber liegt, kommt somit ebenfalls auf dem Beutel zu liegen.

Der zweite Endbereich Z ist in diesem Ausführungsbeispiel ebenfalls mit Klebstoff 41 beschichtet. Alternativ kann auch das Etikett vollflächig mit Klebstoff beschichtet sein. Somit wird auch der zweite Endbereich Z mit dem Rand des Beutels 200 verklebt. Dabei wird die Lasche 50 von dem zweiten Endbereich des Etiketts überdeckt und die Lasche 50 und der zweite Endbereich werden miteinander verklebt.

Durch das Verkleben des zweiten Endbereichs Z mit dem Beutel wird die Lasche 50 zusätzlich fixiert und ein Herausrutschen der Lasche 50 wird somit zuverlässig verhindert. Somit erhält man eine besonders sichere Verbindung zwischen Beutel 200 und Etikett 1.

Nach der Aushärtung des Klebstoffes 40 sind somit Beutel 200 und Etikett 1 fest miteinander verbunden. Die Aushärtung des Klebstoffs kann dabei durch Bestrahlung mit infrarotem oder ultraviolettem Licht aktiviert oder beschleunigt werden. Jegliche weitere Art der Aktivierung oder Beschleunigung einer Aushärtung des Klebstoffes sind ebenso möglich.

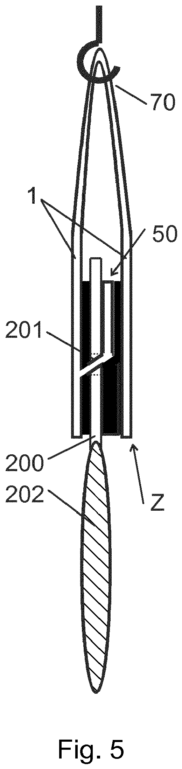

Figur 5 zeigt einen Infusionsbeutel 200 im Querschnitt, an dem ein erfindungsgemäßes Etikett 1 wie oben beschrieben angebracht wurde. Der Infusionsbeutel umfasst dabei im oberen Teil einen Aufhängeabschnitt 200 und im unteren Teil einen Flüssigkeitsreservoir 202. Der obere Aufhängeabschnitt 200 ist dabei so ausgeführt, dass sich darin keine Flüssigkeit befinden kann. Dies kann beispielsweise durch ein geeignetes Verschweißen der Kunststofffolien in diesem Bereich erzielt werden. Die Infusionsflüssigkeit befindet sich daher nur innerhalb des unteren Flüssigkeitsreservoirs 202.

Der Klebstoff 40 des Etiketts kommt hierbei nur im Aufhängebereich 200 mit dem Infusionsbeutel in Berührung, in dem sich keine Flüssigkeit befindet. Der Bereich des Flüssigkeitsreservoirs 202 des Beutels dagegen kommt nicht mit Klebstoff in Berührung, so dass die Gefahr einer Klebstoffmigration zuverlässig ausgeschlossen werden kann. Wie in Figur 5 dargestellt, ist die Lasche 50 durch die ursprüngliche Aufhängeöffnung 201 des Beutels 200 geführt und auf der dem Etikett gegenüberliegenden Seite mit dem umgeklappten Teil des Etiketts verklebt. Auf diese Weise entsteht eine besonders sichere Befestigung des Infusionsbeutels 200 mit dem Etikett 1. Das Risiko, dass sich das Etikett von dem Beutel unbeabsichtigt löst, kann dadurch nahezu ausgeschlossen werden.

Zusammenfassend betrifft die Erfindung ein spezielles Etikett zur Etikettierung von Infusionsbeuteln mit einer Flüssigkeit. Der Beutel wird nur in einem geringen Teilbereich etikettiert, der keine Flüssigkeit enthält. Für eine automatisierbare, sichere Etikettierung besteht das Etikett aus einem speziellen Folienverbund, der es ermöglicht eine Lasche mit speziellen Gelenkseigenschaften auszubilden. Aufgrund der besonderen Befestigungsvariante kann ein unbeabsichtigtes Lösen des Etiketts von dem Infusionsbeutel nahezu ausgeschlossen werden. Ausführungsformen mit zusätzlichen abnehmbaren Teiletiketten sind dabei möglich.

## Patentansprüche

1. Etikett für einen mit einer Flüssigkeit gefüllten Beutel, umfassend
eine erste Folie (10) mit einer zumindest teilweise mit Klebstoff (40) beschichteten Oberseite (11) und einer Unterseite (12); und
eine zweite Folie (20) mit einer Oberseite (21) und einer Unterseite (22); wobei die Unterseite (12) der ersten Folie (10) zumindest teilweise fest mit der Oberseite (21) der zweiten Folie (20) verbunden ist, um einen Folienverbund zu bilden; und
wobei der Folienverbund aus erster Folie (10) und zweiter Folie (20) eine Einstanzung aus einem offenen Linienzug mit zwei Endpunkten (A, B) besitzt, so dass eine streifenförmige Lasche (50, 51) entsteht und die streifenförmige Lasche (50, 51) entlang einer gedachten Linie zwischen den beiden Endpunkten (A, B) mit dem übrigen Folienverbund verbunden ist,
**dadurch gekennzeichnet, dass**
die streifenförmige Lasche (50, 51) eingerichtet ist, durch eine in einem Beutel befindliche Öffnung geführt zu werden, um die streifenförmige Lasche (50, 51) mit einem umgeklappten Teil (Z) des Etiketts zu verkleben, und dass eine weitere erste und eine weitere zweite Einstanzung (70) jeweils aus einem offenen oder einem geschlossenen Linienzug durch die erste und durch die zweite Folie (10, 20) eingerichtet zum Aufhängen des Etiketts vorhanden sind.

2. Etikett nach Anspruch 1, wobei die Unterseite (12) der ersten Folie (10) zumindest teilweise fest mit der Oberseite (21) der zweiten Folie (20) verklebt ist.

3. Etikett nach Anspruch 1 oder 2, wobei die Oberseite (21) der zweiten Folie (20) teilweise mit einer Klebstoff abweisenden Substanz (90) versehen ist und die erste Folie (10) eine weitere Einstanzung (80) umfasst, wobei die erste Folie (10) so auf der auf der zweiten Folie (20) zu liegen kommt, dass sich die weitere Einstanzung (80) im Bereich der Klebstoff abweisenden Substanz (90) befindet.

4. Etikett nach einem der Ansprüche 1 bis 3, wobei die erste Folie (10) eine erste Dicke (d1) besitzt und die zweite Folie (20) eine zweite Dicke (d2) besitzt, wobei die erste Dicke (d1) größer ist als die zweite Dicke (d2).

5. Etikett nach einem der Ansprüche 1 bis 4, das ferner mindestens im Bereich der mit Klebstoff beschichteten Oberseite der ersten Folie (10) mit einem bahnförmigen Material abgedeckt ist.

6. Etikett nach einem der Ansprüche 1 bis 5, das ferner mindestens je eine Einstanzung (60) in der ersten Folie (10) in der Nähe der Endpunkte (A, B) des Linienzugs der Einstanzung der streifenförmigen Lasche (50, 51) umfasst, so dass sich an dem mit dem übrigen Folienverbund verbundenen Ende der Lasche (50, 51) ein Gelenk ausbildet.

7. Etikettenrolle, wobei eine Mehrzahl von Etiketten nach einem der Ansprüche 1 bis 4 auf einem bahnförmigen Material aufgebracht sind und das bahnförmige Material mit der Mehrzahl von Etiketten auf eine Rolle aufgewickelt ist.

8. Verfahren zum Anbringen eines Etiketts nach einem der Ansprüche 1 bis 7 an einem mit einer Flüssigkeit gefüllten Beutel, das die folgenden Schritte umfasst:
Aufnehmen des Etiketts;
Aufstellen der streifenförmigen Lasche (50, 51);
Hindurchführen der streifenförmigen Lasche (50, 51) durch eine Öffnung (201) in dem Beutel (200);
Verkleben mindestens eines Teilbereichs der Oberseite der ersten Folie (10) mit mindestens einem Teilbereich des Beutels; und
Umklappen der aufgestellten streifenförmigen Lasche (50, 51).

9. Verfahren nach Anspruch 8, wobei die Oberseite (11) der ersten Folie (10) mit dem Beutel nur in einem Bereich verklebt wird, in dem sich keine Flüssigkeit befindet.

10. Verfahren nach Anspruch 8 oder 9, wobei die Oberseite (11) der ersten Folie (10) so mit dem Beutel verklebt wird, dass die umgeklappte streifenförmige Lasche (50) von einem Teilbereich der ersten Folie (10) überdeckt wird.

11. Verfahren nach einem der Ansprüche 8 bis 10, das ferner einen Schritt zum Aushärten des Klebstoffs umfasst.

12. Infusionsbeutel, an dem ein Etikett gemäß einem der Ansprüche 1 bis 7 angebracht ist, wobei die streifenförmige Lasche (50, 51) durch eine Öffnung (201) in dem Beutel geführt ist und mit einem umgeklappten Teil des Etiketts verklebt ist.

## Claims

1. A label for a bag filled with liquid, comprising
a first film (10) having an upper side (11) which is coated with an adhesive (40) at least in parts, and a lower side (12); and
a second film (20) having an upper side (21) and a lower side (22);
wherein the lower side (12) of the first film (10) is firmly connected to the upper side (21) of the second film (20) at least in parts in order to form a film compound; and
wherein the film compound made up of the first film (10) and the second film (20) comprises a punched zone in the form of an open polyline having two end points (A, B), so that a strip-shaped tab (50, 51) is produced and the strip-shaped tab (50, 51) is connected to the remaining film compound along an imaginary line between the two end points (A, B),
**characterized in that**
the strip-shaped tab (50, 51) is configured to be guided through an opening provided in the bag in order to glue the strip-shaped tab (50, 51) to a folded part (Z) of the label, and **in that** a further first punched zone and a further second punched zone (70) are provided in each case in the form of an open or a closed polyline by the first and the second film (10, 20) and are configured for hanging the label.

2. The label according to claim 1, wherein the lower side (12) of the first film (10) is firmly glued to the upper side (21) of the second film (20) at least in parts.

3. The label according to claim 1 or 2, wherein the upper side (21) of the second film (20) is provided with an adhesive-repellent substance (90) at least in parts and the first film (10) comprises a further punched zone (80), wherein the first film (10) comes to lie on the second film (20) in such a manner that the further punched zone (80) is located in the region of the adhesive-repellent substance (90).

4. The label according to any of claims 1 to 3, wherein the first film (10) has a first thickness (d1) and the second film (20) has a second thickness (d2), wherein the first thickness (d1) is larger than the second thickness (d2).

5. The label according to any of claims 1 to 4, which is further covered with a web-shaped material at least in the region of the upper side of the first film (10) coated with adhesive.

6. The label according to any of claims 1 to 5, further comprising at least one punched zone (60) in the first film (10) in the vicinity of the end points (A, B) of the polylines of the punched zone of the strip-shaped tab (50, 51), so that a hinge is formed at the end of the tab (50, 51) connected to the remaining film compound.

7. A label coil, wherein a plurality of labels according to any of claims 1 to 4 is applied on a web-shaped material and the web-shaped material together with the plurality of labels is wound up on a coil.

8. A method of applying a label according to any of claims 1 to 7 on a bag filled with liquid, comprising the following steps:
taking the label;
erecting the strip-shaped tab (50, 51);
guiding the strip-shaped tab (50, 51) through an opening (201) in the bag (200);
gluing at least a portion of the upper side of the first film (10) to at least a portion of the bag; and
folding the erected strip-shaped tab (50, 51).

9. The method according to claim 8, wherein the upper side (11) of the first film (10) is glued to the bag only in a region where no liquid is present.

10. The method according to claim 8 or 9, wherein the upper side (11) of the first film (10) is glued to the bag in such a manner that the folded strip-shaped tab (50) is covered by a portion of the first film (10).

11. The method according to any of claims 8 to 10, further comprising a step of curing the adhesive.

12. An infusion bag to which a label according to any of claims 1 to 7 is applied, wherein the strip-shaped tab (50, 51) is guided through an opening (201) in the bag and glued to a folded part of the label.

## Revendications

1. Étiquette pour une poche remplie d'un liquide, comprenant
une première feuille (10) présentant une face supérieure (11) au moins en partie revêtue de colle (40) et une face inférieure (12) ; et
une deuxième feuille (20) présentant une face supérieure (21) et une face inférieure (22) ;
sachant que la face inférieure (12) de la première feuille (10) est au moins en partie fermement reliée à la face supérieure (21) de la deuxième feuille (20) pour former un assemblage de feuilles ; et
sachant que l'assemblage de feuilles composé de la première feuille (10) et de la deuxième feuille (20) possède une perforation composée d'un trait de ligne ouvert avec deux points d'extrémité (A, B) de sorte qu'une languette (50, 51) en forme de lanière se crée et que la languette (50, 51) en forme de lanière soit reliée au reste de l'assemblage de feuilles le long d'une ligne imaginée entre les deux points d'extrémité (A, B),
**caractérisée en ce que**
la languette (50, 51) en forme de lanière est configurée pour être guidée par une ouverture se trouvant dans une poche afin de coller la languette (50, 51) en forme de lanière à une partie rabattue (Z) de l'étiquette, et **en ce qu'**une première perforation supplémentaire et une deuxième perforation supplémentaire (70) configurées respectivement à partir d'un trait de ligne ouvert ou un trait de ligne fermé à travers la première et la deuxième feuille (10, 20) sont prévues pour l'accrochage de l'étiquette.

2. Étiquette selon la revendication 1, sachant que la face inférieure (12) de la première feuille (10) est au moins en partie fermement collée à la face supérieure (21) de la deuxième feuille (20).

3. Étiquette selon la revendication 1 ou 2, sachant que la face supérieure (21) de la deuxième feuille (20) est pourvue en partie d'une substance anti-adhésive (90) et la première feuille (10) comprend une perforation (80) supplémentaire, sachant que la première feuille (10) vient reposer de telle manière sur la deuxième feuille (20) que la perforation (80) supplémentaire se trouve dans la zone de la substance anti-adhésive (90).

4. Étiquette selon l'une des revendications 1 à 3, sachant que la première feuille (10) présente une première épaisseur (d1) et la deuxième feuille (20) présente une deuxième épaisseur (d2), sachant que la première épaisseur (d1) est plus grande que la deuxième épaisseur (d2).

5. Étiquette selon l'une des revendications 1 à 4, laquelle est en outre, au moins dans la zone de la face supérieure revêtue de colle de la première feuille (10), recouverte d'un matériau en forme de bande.

6. Étiquette selon l'une des revendications 1 à 5, laquelle comprend en outre au moins respectivement une perforation (60) dans la première feuille (10) à proximité des points d'extrémité (A, B) du trait de ligne de la perforation de la languette (50, 51) en forme de lanière de telle sorte qu'une articulation se forme à l'extrémité de la languette (50, 51) qui est reliée au reste de l'assemblage de feuilles.

7. Rouleau d'étiquettes, sachant qu'une pluralité d'étiquettes selon l'une des revendications 1 à 4 sont apposées sur un matériau en forme de bande et le matériau en forme de bande comportant la pluralité d'étiquettes est enroulé sur un rouleau.

8. Procédé d'apposition d'une étiquette selon l'une des revendications 1 à 7 sur une poche remplie d'un liquide, comprenant les étapes suivantes :
prise de l'étiquette ;
dressage de la languette (50, 51) en forme de lanière ;
guidage de la languette (50, 51) en forme de lanière à travers une ouverture (201) dans la poche (200) ;
collage d'au moins une zone partielle de la face supérieure de la première feuille (10) à au moins une zone partielle de la poche ; et
rabattage de la languette (50, 51) en forme de lanière dressée.

9. Procédé selon la revendication 8, sachant que la face supérieure (11) de la première feuille (10) est collée à la poche uniquement dans une zone dans laquelle aucun liquide ne se trouve.

10. Procédé selon la revendication 8 ou 9, sachant que la face supérieure (11) de la première feuille (10) est collée de telle façon à la poche que la languette (50) en forme de lanière rabattue soit recouverte par une zone partielle de la première feuille (10).

11. Procédé selon l'une des revendications 8 à 10, lequel comprend en outre une étape de durcissement de la colle.

12. Poche de perfusion sur laquelle est apposée une étiquette selon l'une des revendications 1 à 7, sachant que la languette (50, 51) en forme de lanière est guidée par une ouverture (201) dans la poche et collée à une partie rabattue de l'étiquette.
